# EUROPEAN PATENT SPECIFICATION

(11) **EP 2 525 726 B1**
(45) Date of publication and mention of the grant of the patent: **04.05.2016**
(21) Application number: 11712327.3
(22) Date of filing: 20.01.2011
(51) Int. Cl.: A61B 17/17, A61B 17/80

(54) **BONE FRACTURE FIXATION MEANS**
FIXATIONSVORRICHTUNG FÜR KNOCHENBRÜCHE
MOYEN DE FIXATION POUR FRACTURES D'OS

(30) Priority: 11.10.2010 GB 201017027; 20.01.2010 GB 201000829
(43) Date of publication of application: 28.11.2012
(73) Proprietor: Kumar, Deepak, Doncaster, DN4 6EH (GB)
(72) Inventor: Kumar, Deepak, Doncaster, DN4 6EH (GB)
(86) International application number: PCT/IB2011/050262
(87) International publication number: WO 2011/089564

(56) References cited:
- WO-A1-2009/121144
- DE-A1-102005 043 281
- US-A1- 2005 085 818
- US-A1- 2005 124 990
- US-A1- 2006 173 458
- US-A1- 2009 157 086
- US-A1- 2009 177 239
- US-A1- 2009 228 047

## Description

### BACKGROUND

The invention relates to fixation of fractures of distal radius but several of its features may be used in fixation of many fracture especially those close to the joint (juxta-articular). Displaced juxta-articular or intra-articular fractures and many displaced metaphyseal fractures are now commonly treated by operative method (open reduction and internal or external fixation).

Relatively less complex fractures are treated by closed manipulation and stabilisation with K-wires. For more complex fractures especially displaced intra-articular fractures, open reduction and internal fixation with plate and screws has become choice of most surgeons because it allows direct, accurate reduction and early mobilisation of joint. For very few fractures where anatomical reduction is not necessary, intra-medullary implants such as nails are used but they have not gained popularity because such fractures can be successfully treated with less expensive and simpler procedures using k-wires. Moreover, nails are not suitable for the commonly encountered juxta- or intra-articular fractures.

K-wiring, although simpler to use, has several disadvantages. Commonly, it is left exposed out of skin and therefore subjects to the risk of infection. It also requires plaster immobilisation following the procedure as it provides a weak stabilisation on its own.

There are various plating systems available today for fixation of juxta-articular fractures of distal radius. The plates applied on volar aspect with locking screws are very popular.

After a good surgical exposure, precise placement of the plate in the correct position is the most important first step. The quality of bone is often suboptimal especially in the elderly, who sustain the fractures of distal radius most commonly.The best quality of bone is in the subchondral region where screws can have good purchase. This region is very close to the joint, therefore many surgeons trying not to enter into the joint with their screws end up too far proximal in not so good bone and hence, risk failure of fixation. Most plates have an oblong hole in the part that is applied onto the diaphyseal fragment to allow adjustment in plate position. This may offer adjustment after initial fixation to the diaphyseal fragment with one screw through this hole. There are plates that have another transverse oblong hole but again that is in the part that is applied onto the diaphyseal fragment and require fixation to diaphyseal fragment first. Adjustments longer than the length of the oblong hole, a transverse translation of the plate or in longitudinal direction after insertion of screw in the transverse oblong hole are not possible without making another drill hole for another screw. This can certainly cause unnecessary additional weakness in the bone. Furthermore, improvement in fracture reduction becomes extremely difficult if not impossible after fixation to the diaphyseal fragment especially the rotational displacements (supination or pronation of the distal fragment). Therefore, many surgeons choose to do the fixation to the juxta-articular fragment first. This allows them to reduce the displaced juxta-articular fragment better as the attached plate provides better hold on the displaced short fragment. In the technique that involves juxta-articular fixation first, surgeons do not have means of adjustment in plate positioning with most plates currently available. The one plate known to author that allows adjustment over the juxta-articular part is restricted to transverse direction. This requires insertion of a screw in a transversely aligned oblong hole. If the position in longitudinal direction is found to be less than satisfactory after insertion of screw in the said transverse oblong hole, the procedure has to be started all over again. Inaccurate placement of the plate leads to prominence of the plate and screws, irritation to the adjacent soft tissue, attrition and rupture of tendons and loss of fixation of fracture. Fine adjustment to the initial placement of the plate to achieve accurate positioning of the plate is highly desirable and is the subject of this invention.

### SUMMARY OF INVENTION

The invention constitutes of a system (a set of devices) for facilitating the use of a bone fracture fixation plate, comprising means to facilitate adjustment in positioning of the plate on the juxta-articular fragment prior to fixation on the diaphyseal fragment.

The present invention relates to a system for facilitating the use of a bone fracture fixation plate as claimed hereafter. Preferred embodiments of the invention are set froth in the dependent claims.

The system constitutes of a plate with its plate specific companion drill guiding jig, a jig and plate specific drill sleeve and an eccentric hole plate positioner for which we claim novelty. The system also constitutes of screws of various types and pins for which we do not claim novelty.
a) The plate has a *juxta-articular part* with more than one row of threaded holes, a *neck,* a *shaft or diaphyseal part, a pin and a tail. The juxta-articular part* of the plate is twisted in relation to the diaphyseal part. The plate can be further contoured to the anatomy of the surface of the juxta-articular bone during the procedure. The plate has either a group of linked slots or an arrangement of aperture in the plate comprising of at least four holes in the middle of juxta-articular part for temporary fixation of the plate to the juxta-articular fragment with one positioning wire and subsequent adjustment in position of the plate in longitudinal, transverse and oblique directions. Once the plate is positioned precisely to the correct place it can be further secured by inserting pins in the pin holes in the juxta-articular part of the plate before insertion of the definitive screws. There are threaded holes in the extensions to the juxta-articular end of the plate and these extensions can be individually bent and readily detached from the plate if required. *The neck part* has a threaded hole with a recessed collar for the drill guiding jig. *The diaphyseal part* of the plate has threaded oblique holes directed from juxta-articular side and outer or superficial surface (surface away from bone) to diaphyseal side and inner or deep surface (close to bone) for screws. *The pin part* is integral to and extends from the diaphyseal end and has sharpening at the end remote from the plate to facilitate insertion into the cortex of the bone. *The tail part* is integral to and extends from the juxta-articular end of the plate. This can be held in a chuck or a power tool and the pin part penetrates the cortex of the bone like a drill bit. The pin part is bent where it comes out of the bone after said insertion of the pin into the cortex. The said tail is suitable to be inserted in a hole in the region of the outer end of a bone-fixing slotted head screw and is suitable for the tail beyond the screw to be cut off before the tail is fixed in place in such a slot. The pin part and tail part can be cut off and the rest of the plate can be used for fracture fixation.
b) The drill guiding jig is plate specific and is configured to sit on a juxta-articular part of the plate. It is attached to the plate with a screw through a hole, which has a proud collar on the under surface to fit into the recessed collar on the plate. The drill guiding jig has either the group of linked slots or the arrangement of aperture with at least twee four holes corresponding to those into the plate. The jig also has other holes corresponding to the holes in the plate except for those in the bendable and detachable extensions to the juxta-articular end of the plate.
c) The drill sleeve that is specific to the above two, lines the apertures in the jig to protect the jig from the drill bit that is guided by these apertures, can lock into the threaded holes of the plate, has scales over its body to indicate how far into the bone a drill bit passing through the sleeve has penetrated and this can be seen from four sides and has means to lock the plate and the jig to a positioning and/or fixing member in an aperture in the plate and the jig during adjustment in position of the plate.
d) Eccentric hole plate positioner allows further controlled, fine adjustment in plate position by rotating over a positioning and/or fixing member in an aperture in the plate. The eccentric hole of the plate positioner allows to move the plate in a controlled manner in the desired direction when rotated and has means to temporarily fix the plate to the bone.

### ADVANTAGES

The plate of this system has following advantages over other plates currently available.
1. The adjustment apertures (a group of linked slots or a group of holes) towards the centre of the juxta-articular part allows adjustment in the positioning of the plate transfixed to the juxta-articular fragment of the fractured bone with a positioning wire that remains in its initial position relative to the bone whilst position of the plate is adjusted in longitudinal, transverse or oblique directions, prior to fixation to the diaphyseal fragment. This facilitates precise placement of the plate in the desired position on the bone on the juxta-articular side of the fracture without need for removing and re-inserting any fastener or pin into the bone.
2. The eccentric hole plate positioner allows controlled, fine adjustment in the positioning of the plate by rotating over a positioning wire inserted into the bone through an aperture in the plate.
3. This system allows the surgeon to lock the drill sleeve or the eccentric plate positioner to the positioning wire thus temporarily securing the position of the plate or plate-jig assembly and hence, providing the surgeon with freedom from manually holding handle of another instrument to keep the plate in place while the position is checked by x-ray or during insertion of definitive screws. The limb can be moved to obtain better orthogonal views without fear of losing the position.
4. The pin part, an extension from the diaphyseal end can penetrate bone with minimal soft tissue exposure and allows for longer hold into the bone.
5. The pin part and the tail part can be cut off and the rest can be used as a standard plate.
6. The obliquely placed screw holes in the diaphyseal part of the plate allow screws to be inserted only in a predetermined direction from juxta-articular side and outer surface (surface away from bone) to diaphyseal side and inner surface (close to bone). With the screws in this configuration, the plate is wedged between screws and bone rather than pushed away from the bone by the forces of the tendons across the fracture.
7. The measurement of depth of drill bit into the bone can be seen from all directions.

### INTRODUCTION TO DRAWINGS

The invention now will be described by referring to the accompanying Figures.
Figure 1 shows the plate in front profile with adjustment slots
Figure 2. shows the plate in front profile with adjustment holes
Figure 3 shows the plate in side profile
Figure 4 shows the plate in an end profile from the juxta-articular end
Figure 5 shows the drill guiding jig in front profile
Figure 6 shows the drill guiding jig in side profile
Figure 7 shows the drill sleeve in front, side and top end profiles

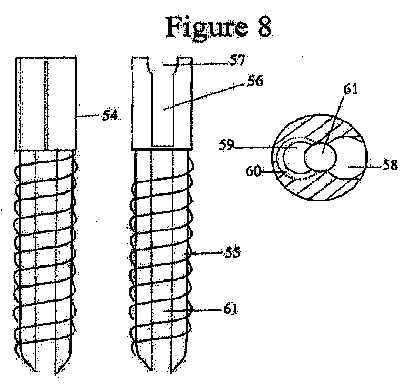

Figure 8 shows the slotted head top loading screw in front, side and top end profiles
Figure 9 shows the eccentric hole plate positioner in front, top and bottom end profiles

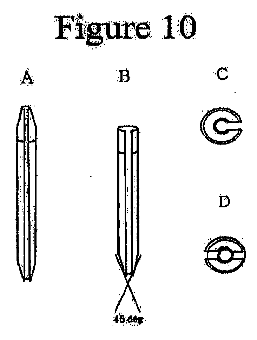

Figure 10 shows both members of the two piece pin bender. A, is front profile of inner member, B is front profile of outer member, C is top end profile when both members are together in an open position and D is top end profile when both members are together in a closed position
Figure 11 shows the front view of juxta-articular radius fracture stabilised with a plate
Figure 12 shows the side view of juxta-articular radius fracture stabilised with a plate

### DETAILED DESCRIPTION

A juxta-articular bone fracture fixation plate is illustrated and described in US 2009/157086A1.

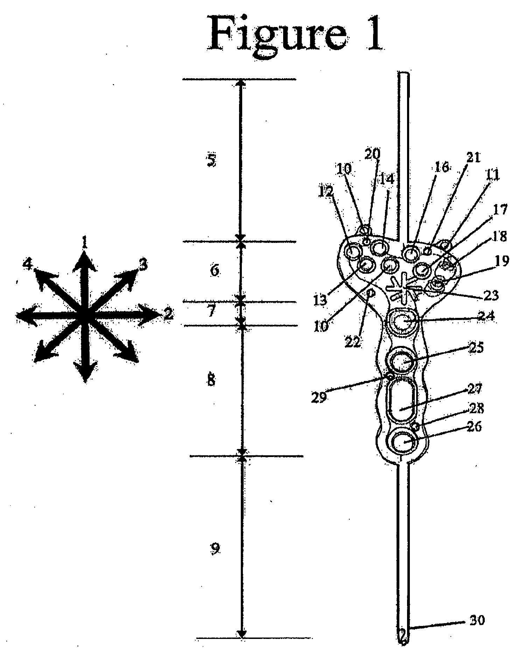

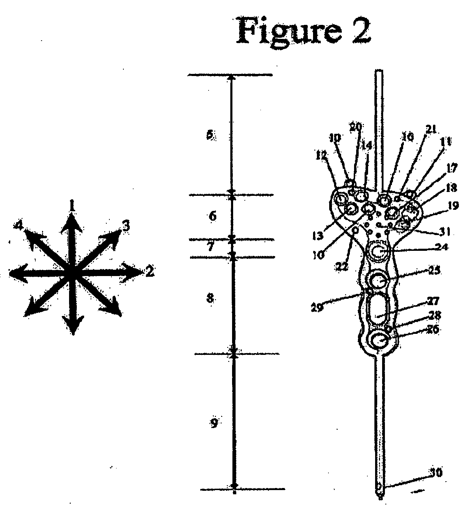

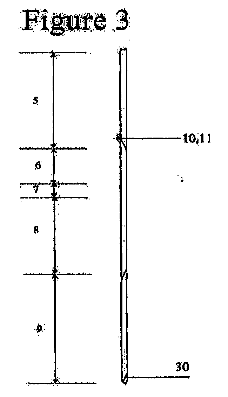

Means 5-9, Figure 1, 2 & 3 for facilitating the use of a bone fracture fixation plate 5-9, comprising means 23, 31, Figures 1 & 2, to facilitate adjustment in positioning of the plate 5-9 and/or means 30, of part 9 of the plate 5-9, Figure 1,2 & 3 to facilitate insertion into the cortex of the bone 67 and/or part 5 of the plate 5-9, Figure 1, 2 & 3 to facilitate holding the plate in a power tool and/or insertion into the hole 57,58, of the slotted head screw 54-61 to facilitate stabilisation of a fragment that could not be stabilised by screws in the plate 5-9 are described below with reference to the above mentioned drawings.

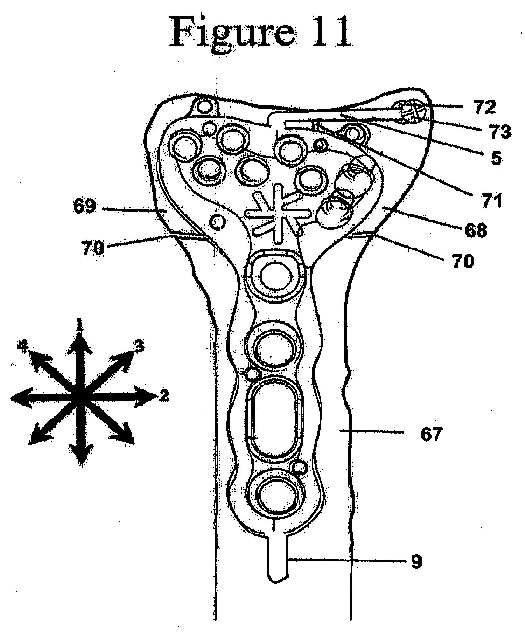

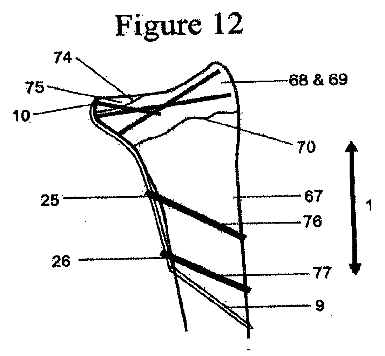

The means 5-9, comprising a bone fracture fixation plate positioning means 23, 31 for facilitating useful, controlled and stable adjustment of the position of the plate 5-9 on a long bone 67, Figures 11 & 12, on the juxta-articular side 68, 69 of a fracture 70, during fixation on that side and before fixation or positioning to the bone on the diaphyseal side 67, such positioning means 23, 31 being adapted to facilitate controlled adjustment of position of the plate 5-9 on the bone in longitudinal directions (parallel to the long axis of the limb 1, Figure 1,2,5,6,11,12), transverse directions (that is perpendicular to the long axis, 2, Figure 1,2,4,5,11) and oblique directions (that is any direction non-parallel to the long axis, 3,4, Figure 1,2,5,11);

Means 5-9 which comprise the plate 5-9 is shown in Figures 1 to 4.

The means 6 has a slot means 23 comprising a cruciform slot arrangement 23 as shown in Figure 1.

Alternatively, the means 6 has an aperture means 31 comprising an arrangement of holes 31 that comprises of at least four holes is shown in Figure 2.

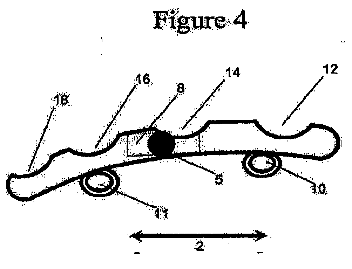

The means 5-9 comprising a bone fracture fixation plate 5-9 has a juxta-articular part 6 and a elongate diaphyseal part 8 connected together and twisted relative to one another is shown in Figure 4.

The means 6 has extensions 10, 11, Figures 1,2 & 3 on its juxta-articular end and these extensions 10, 11 comprise means adapted to guide and/or hold positioning and/or fixing member/s 80 inserted through the extensions 10, 11 into parts of the bone 68, 69 as shown in Figures 11 & 12.

Means 25, 26 Figures 1 and 2, comprise of guide means 25, 26 that are directed obliquely towards the bone 67 in a direction away from_the fracture 70, for example from juxta-articular side and outer surface (away from bone) to diaphyseal side and inner (close to bone)' and fixing members 76, 77, when inserted into the bone 67 through these guide means 25, 26 follow the above said direction as shown in Figure 12.

The jig means 43 has a slot means 40 comprising of a cruciform slot arrangement 40 corresponding to the slot arrangement 23 on means 6 of the plate 5-9.

Alternatively, the jig means 43 can have an aperture means comprising an arrangement of holes similar to 31 that comprises of at least four holes.

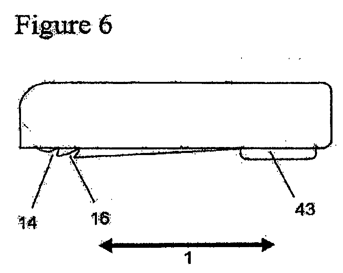

The jig means 43 has a prominent means 44 on its undersurface as shown in Figure 6, configured to sit into a recession around means 24 of the juxta-articular part 6 of the plate 5-9.

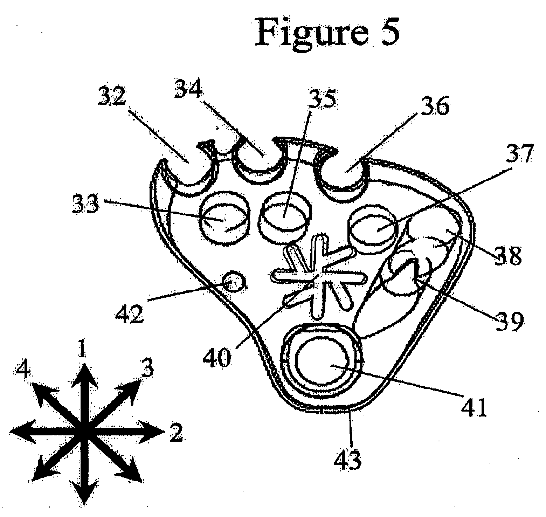

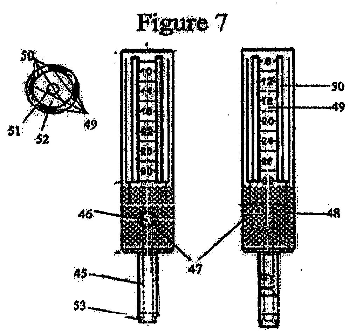

The nozzle part 45 of the sleeve means 48 that lines aperture means 32-39, Figure 5 in the jig means 43 to protect the jig means 43 from drill bits that they guide is shown in Figure 7.

The sleeve means 48, comprises means 46 (a side screw hole) and 47 (threads for a side screw), Figure 7 to lock to the jig means 43 and plate 5-9 to a positioning member in aperture means 40 and 23 or 31.

The sleeve means 48 comprises 4 scale means 49 adapted to indicate through the 4 windows 50 how far into the bone 68, 69 a drill bit passing through the hole 51 in the sleeve means 48 has penetrated is shown in Figure 7.

Means 48 in which the scale means 49 are adapted to be viewed from all sides is shown in the 'top plan' part of Figure 7.

The drill guiding jig 43 is adapted to be fixed in relation to the plate 5-9 on its neck part 7, Figures 1 and 2. This is done by a non-integral fixing member passing through an aperture means 41, Figure 5 in the jig 43 and engaging in a corresponding aperture 24 in the neck part 7 of the plate 5-9 Figures 1 and 2.

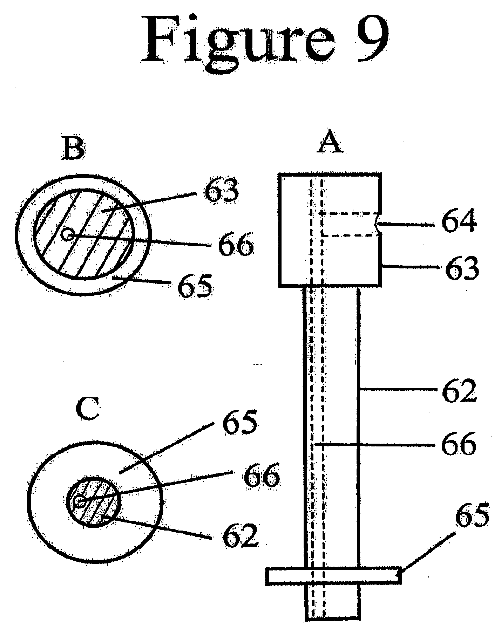

The means 5-9 with the help of a eccentric plate positioner means 62-66, Figure 9 allows further controlled fine adjustment in the positioning of the plate 5-9 in all directions in the same plane as 1,2,3 & 4 but not limited to 1, 2, 3 & 4.

## Claims

1. A bone fracture fixation system, for facilitating useful, controlled and stable adjustments in positioning a bone fracture fixation plate comprising: *a bone fracture fixation plate (6-8)* that has a juxta-articular part (6,7) with bendable detachable extensions (5,10,11) and a diaphyseal part (8) with or a bendable, detachable extension (9) and an inner surface close to the bone and outer surface away from the bone, *a drill guiding jig (43)* assembled to the outer surface of the juxta-articular part (6,7) of the plate, both having plurality of apertures of at least two dimensions, one with larger dimension (12-19, 24-27, 32-39, 41) with threads in the holes in the plate for fasteners with threaded head and without threads in the holes in the drill guiding jig and the other with smaller dimension (20-22,28,29,42) for pins located anywhere but usually near the margins for temporarily securing the plate to the bone, and a drill sleeve (45-53), **characterised in that**
the plate at least one has additional adjustment aperture (23,31) towards the centre of the juxta-articular part (6,7) away from the margins for a positioning wire that transfixes the plate or through one of the adjustment apertures to a juxta-articular fragment (68,69) of a fractured bone and remains in its initial position relative to the bone whilst position of the plate (6-8) is adjusted in longitudinal directions (that is parallel to the long axis of the limb, 1) and/or transverse directions (that is perpendicular to the long axis, 2) and/or oblique directions (that is any direction between longitudinal and transverse axes, 3,4), prior to fixation to a diaphyseal fragment (67) wherein the system further comprises an eccentric plate positioner(62-66) and a positioning wire wherein the positioning wire is eccentrically located in the eccentric plate positioner (62-66) by means of a hole (66) and the plate (5-9) is finely adjusted to a desired position relative to the bone by rotating the eccentric plate positioner (62-66) about the positioning wire.

2. The bone fracture fixation system of claim 1, wherein the plate and the jig have a plurality of position adjusting apertures, wherein the position adjusting aperture is long and narrow like a slot.

3. The bone fracture fixation system of claim 2, wherein the slot of the plate and the jig is tri-radiate.

4. The bone fracture fixation system of claim 2, wherein the slot of the plate and the jig is cruciform.

5. The bone fracture fixation system of claim 4, wherein the slot of the plate and the jig has additional branches to allow position of the plate to be adjusted in directions intermediate to the four branches.

6. The bone fracture fixation system of claim 4, wherein the slot of the plate and the jig has additional branches emanating from the four branches of the cruciform.

7. The bone fracture fixation system of claim 1, wherein the position adjusting apertures of the plate and the jig comprise of an arrangement of 4 or more holes.

8. The bone fracture fixation system of claim 1, wherein the plate has a diaphyseal part wherein the aperture/apertures (25,26) in the diaphyseal part (8) is/are directed obliquely **characterised in that** this obliquity is from outer surface to inner surface and from juxta-articular end to diaphyseal end, to guide the fasteners (76,77) in the same direction so that they remain parallel and when forces of the tendons across the fracture pull the juxta-articular fragment (68,69) and the attached plate (6-8) towards the diaphyseal fragment (67), the plate is wedged between these fasteners and the diaphyseal fragment and more firmly applied to the surface of diaphyseal bone.

9. The bone fracture fixation system of claim 1, wherein the drill sleeve has 4 windows and scale marking on the parts intervening the windows and marking on the drill can be visualised through these 4 windows (50) to determine how far drill has traversed into the bone with the help of scales marked on 4 sides (49).

10. The bone fracture fixation system of claim 9, wherein the drill sleeve has a threaded side hole (46) for a fastener so that when the drill sleeve is mounted over a positioning wire transfixing the plate to the bone and the fastener in the side hole is tightened onto the positioning wire while the drill sleeve is pressed down firmly onto the plate, their position relative to each other and bone is held securely.

11. The bone fracture fixation system of claim 1, wherein the eccentric plate positioner (62-66) has an eccentric cannulation (66), a flange (65) close to the bottom end and a threaded side hole (64) for a fastener near the top end so that when the positioner is mounted over a pin positioning wire transfixing the plate to the bone through one of the larger dimension holes and the positioner is rotated inside the hole over the positioning wire it can facilitate finer adjustments in the positioning of the plate and when the fastener near the top end is tightened onto the positioning wire while the positioner is pressed down firmly onto the plate, their position relative to each other and bone is held securely.

## Patentansprüche

1. Ein Knochenbruch Fixationssystem, zur Erleichterung nützlich, kontrollierte und stabile Anpassungen bei der Positionierung einer Knochenbruch Fixationsplatte, mit:
ein Knochenbruch FIxationsplatte (6-8), das hat eine juxta-Gelenktell (6,7) mit biegbar, abnehmbare Erweiterungen [5,10,11] und einem diaphysären Tell (8) mit einem biegsamen, abnehmbare Verlängerung (9) und eine innere Oberfläche nahe an den Knochen und der Außenfläche weg von dem Knochen, eine Bohrerführungs Einspannvorrichtung (43) montiert an der Außenfläche des juxta-Gelenkteil (6,7) der Platte, die beide Vielzahl von Öffnungen von zumin mindestens zwei Dimensionen, eine mit größerer Abmessung (12-19, 24-27, 32-39, 1) mit einem Gewinde in die Löcher in der Platte für Befestigungselemente mit Gewindekopf und ohne Gewinde In die Löcher in der Bohrerführungs Einspannvorrichtung und das andere mit kleineren Dimension (20-22,28,29,42) für Pins überall, aber in der Regel in der Nähe der Kanten angeordnet um vorübergehend die Platte an den Knochen zu sichern, und einer Bohrhülse (45-53)
**dadurch gekennzeichnet**,
weist die Platte mindestens eine zusätzliche Anpassungsöffnung (23,31) in Richtung der Mitte des juxta-articular Tell (6,7) von den Rändern entfernt für eine Positionierungsdraht, dass transfixes die Platte durch eine der Anpassung Öffnungen an einen juxta-Gelenk Fragment (68,69) eines gebrochenen Knochens und bleibt in seiner Ausgangsposition relativ zu dem Knochen während Änderung der Position der Platte (6-8) In Längsrichtung eingestellt (dh parallel zur Längsachse das Giled, 1) und / oder Querrichtung (dh senkrecht zur Längsachse, 2) und / oder schrägen Richtungen (dh jede Richtung zwischen Längs- und Querachsen, 3,4) vor der Fixierung auf ein diaphysären Fragment (67) wobei das System ferner aufweist eine exzentrische Platte Positionierer (62-66) und eine Positionierungsdraht, worin die Positionierung Draht wird exzentrisch gelegen In der exzentrischen Positionierer (62-66) durch das Loch (66) und der Platte (5-9) ist fein eingestellt durch zu drehen Exzenterscheibe Positionierer (62-66) herum Positionierungsdraht in einer gewünschten Position relativ zu dem Knochen.

2. Die Knochenbruchfixierungssystem nach Anspruch 1, wobei die Platte und die Einspannvorrichtung eine Mehrzahl von Positionseinstellungs Öffnungen aufweisen, wobei die Position Blenden Einstellen lang ist und wie ein Schlitz verengen.

3. Die Knochenbruchfixierungssystem nach Anspruch 2, wobei der Schlitz der Platte und der Einspannvorrichtung tri-abstrahlen ist

4. Die Knochenbruchfixierungssystem nach Anspruch 2, wobei der Schlitz der Platte und der Einspannvorrichtung kreuzförmigem ist.

5. Die Knochenbruchfixierungssystem nach Anspruch 4, worin der Schlitz der Platte und die Vorrichtung hat zusätzliche Zweige zu Positionswechsel ermöglichen der Platte In Richtungen zwischen den vier Zweigen.

6. Die Knochenbruchfixierungssystem nach Anspruch 4, wobei der Schlitz der Platte und der die Bohrführung jig Zweige von den vier Zweige der Kreuzform ausgeht hat.

7. Die Knochenbruchfixierungssystem nach Anspruch 1, wobei die Öffnungen für Einstellung der Position der Platte und der die Bohrführung jig umfassen eine Anordnung von 4 oder mehr Löchern.

8. Die Knochenbruchfixierungssystem nach Anspruch 1, wobei die Platte einen diaphysären Abschnitt aufweist, wobei die Öffnung / Öffnungen (25,26) Im diaphysären Teil (8) ist / sind schräg, **dadurch gekennzeichnet, dass** die Richtung der Schiefe von der ist Außenfläche zur Innenfläche und von der Juxtaarticular Ende zum diaphysären Ende (76,77) Befestigungselemente in die gleiche Richtung zu leiten, so dass sie parallel bleiben und, wenn Kräfte der Sehnen ziehen juxta-artikuläre Fragment (68,69) und das befestigt Platte (6-8) in Richtung des diaphysären Fragment (67) wird die Platte zwischen diesen Verbindungselementen und dem diaphysären Fragment vorkellt und angewandter mehr fest auf der Oberfläche des diaphysären Knochen.

9. Die Knochenfrakturfixationssystem nach Anspruch 1, wobei die Bohrhülse hat 4 Fenster und die Teile zwischen den Fenstern haben Lineale [49], kann die Markierung auf die Bohrkrone durch diese 4 Fenster (50) sichtbar gemacht werden, um zu bestimmen, wie weit der Bohrer hat sich durch in den Knochen gegangen

10. Die Knochenfrakturfixationssystem nach Anspruch 9, wobei die Bohrhülse einen mit Gewinde versehenen Seitenloch (46) für ein Befestigungselement aufweist, so dass, wenn die Bohrhülse über einen Positionierungsdraht angebracht wird, feststecken die Platte an den Knochen und das Befestigungselement in der Seitenloch auf zu des Positionierungsdraht angezogen, während die Bohrhülse wird, relativ zueinander ihre Position fest auf die Platte gedrückt und Knochen sicher gehalten wird.

11. Die Knochenbruchfixierungssystem nach Anspruch 1, wobei die exzentrische Platte Positionierer (62-66) hat einen exzentrischen Kanüllerung (66), einen Flansch (65) nahe dem unteren Ende und einem Gewindeseitenloch (64) für ein Befestigungselement nahe dem oberen Ende, so dass, wenn der Stellungsregler über einen Positionierungsdraht feststecken der Platte an den Knochen durch eine der größeren Abmessung Löcher angebracht ist und der Positionierer wird in das Loch über den Positionierungsdraht gedreht es feinere Einstellungen in der Positionierung der erleichtern Platte und wenn das Befestigungselement in der Nähe des oberen Endes auf den Positionierungsdraht angezogen wird, während der Positionier fest auf die Platte gedrückt wird, ihre Position relativ Zueinander und Knochen sicher gehalten wird.

## Revendications

1. Système de fixation de fracture osseuse, destiné faciliter les ajustements utiles, contrôlé et stable dans le positionnement d'une plaque de fixation de fracture osseuse, comprenant:
*une plaque de fracture osseuse de fixation* (6-8) qui a une partie juxta-articulaire (6,7) avec extensions détachable pliables, (5,10,11) et une partie diaphysaire [8] avec une extension détachable pliables (9) et une surface intérieure près de l'os et la surface externe loin de l'os, un *gabarit pour guider un foret* (43) assemblés à la surface extérieure de la partie juxta-articulaire (6,7) de la plaque, les deux ayant plusieurs ouvertures d'au moins deux dimensions, l'une de plus grande dimension (12-19,24-27,32-39, 41) avec le filet de vis dans les trous de la plaque pour les fixations à tête filetée et sans fil de vis dans les trous dans le gabarit pour guider un foret et l'autre avec plus petite dimension (20-22,28,29,42) pour les broches situées partout mais généralement près des marges pour fixer temporairement la plaque à l'os, *et un manchon de forage* (45-53),
**caractérisé en ce que**
la plaque comporte au moins une ouverture d'adaptation supplémentaire (23,31) vers le centre de la partie juxta-articulaire (6,7) à l'écart des marges pour un fil de positionnement qui transperce la plaque à travers l'une des ouvertures d'ajustement à une fragment juxta-articulaire (68,69) d'un os fracturé et reste dans sa position Initiale par rapport à l'os tandis que la position de la plaque (6-B) est réglée dans le sens longitudinal (qui est parallèle à l'axe longitudinal de la membre, 1) et / ou dans le sens transversal (qui est perpendiculaire à l'axe long, 2) et / ou des directions obliques (soit toutes les directions entre les axes longitudinaux et transversaux, 3,4), avant la fixation à un fragment diaphysaire (67), dans lequel le système comprend en outre un dispositif de positionnement excentrique de la plaque (62-66) et un fil de positionnement dans lequel le fil de positionnement est situé de façon excentrée dans le dispositif de positionnement excentrique de la plaque (62-66) par l'intermédiaire du trou (66) et la plaque (5-9) est finement ajustée à une position désirée par rapport à l'os en faisant tourner le dispositif de positionnement excentrique de la plaque (62-66) autour du fil de positionnement.

2. Système de fixation de fracture osseuse selon la revendication 1, dans lequel la plaque et le modèle comporte une pluralité d'ouvertures pour le réglage de positions, dans lequel l'ouverture pour le réglage de la position est long et étroit, comme une fente.

3. Le système de fixation de fracture osseuse selon la revendication 2, dans lequel la fente de la plaque et le gabarit est le tri-rayonnent.

4. Le système de fixation de fracture osseuse selon la revendication 2, dans lequel la fente de la plaque et le gabarit est cruciforme.

5. Système de fixation de fracture osseuse selon la revendication 4, dans lequel la fente de la plaque et le gabarit a des branches supplémentaires pour permettre la position de la plaque d'être ajustée dans les directions intermédiaire aux quatre branches.

6. Système de fixation de fracture osseuse selon la revendication 4, dans lequel la fente de la plaque et le gabarit a des branches supplémentaires issus des quatre branches de la croix.

7. Le système de fixation de fracture osseuse selon la revendication 1, dans lequel la position de réglage des ouvertures de la plaque et le bâti comprend un agencement de trous de 4 ou plus.

8. Le système de fracture osseuse de fixation selon la revendication 1, dans lequel la plaque comprend une partie diaphysaire dans laquelle l'ouverture / les ouvertures (25,26) Aucours partie diaphysaire (8) est / sont dirigés obliquement **caractérisé en ce que** l'obliquité est de la surface extérieure à la surface intérieure et de la bout juxta-articulaire à bout diaphysaire, pour guider les éléments de fixation (76,77) dans la même direction, de sorte qu'ils restent parallèles et, lorsque les forces des tendons à travers la fracture tirent le fragment juxta articulaire (68,69) et la plaque fixée (6-8) sur le fragment d'arbre (67), la plaque est entre coincèr ces éléments de fixation et le fragment diaphysaire et plus fortement appliqués sur la surface de l'os diaphysaire.

9. Le système osseux de fixation de fracture selon la revendication 1, dans lequel le manchon de forage a 4 fenêtres et une échelle de notation sur les parties entre les fenêtres et le marquage sur la perceuse peut être visualisée à travers ces 4 fenêtres (50) afin de déterminer dans quelle mesure forage a traversé en l'os à l'aide d'écailles marquées sur les 4 côtés (49).

10. Le système osseux de fixation de fracture selon la revendication 9, dans lequel la douille de guidage comporte un trou latéral fileté (46) pour un élément de fixation de telle sorte que lorsque le manchon de forage est monté sur un fil de positionnement transperçant la plaque sur l'os et l'élément de fixation sur le côté le trou est serré sur le fil de positionnement tandis que la douille de guidage est pressé fermement sur la plaque, leur position par rapport à l'autre et de l'os est maintenue en toute sécurité.

11. L'os Système de fixation de fracture selon la revendication 1, dans lequel le positionneur excentrique de la plaque (62-66) comporte une canulation d'excentrique (66), une bride (65) proche de l'extrémité inférieure et un trou latéral fileté (64) pour un attache près de l'extrémité supérieure de sorte que, lorsque le positionneur est monté sur un fil de positionnement transperçant la plaque sur l'os à travers l'un des plus grands trous de dimension et de positionneur est mis en rotation à l'intérieur du trou sur le fil de positionnement, il peut faciliter des ajustements plus fins dans le positionnement de la et la plaque lorsque l'attache près de l'extrémité supérieure est serrée sur le fil de positionnement tandis que le positionneur est pressée fermement sur la plaque leur position par rapport à l'autre et de l'os est maintenue en toute sécurité.
